# EUROPEAN PATENT APPLICATION

(11) **EP 1 041 150 A1**
(43) Date of publication of application: **04.10.2000**
(21) Application number: 99105659.9
(22) Date of filing: 19.03.1999
(51) Int. Cl.: C12N 15/52, C12N 9/00, C12P 21/02

(54) **Hybrid synthetase, fungus/bacterium strain, hybrid peptide and compositions containing same**

(71) Applicant: Gesellschaft für Biotechnologische Forschung mbH (GBF), D-38124 Braunschweig (DE)
(72) Inventor: Timmis, Kenneth Nigel, 38124 Braunschweig (DE); Golyshin, Peter, 38124 Braunschweig (DE); Yakimov, Micheal, 38124 Braunschweig (DE)
(74) Representative: Boeters, Hans Dietrich, Dr.

(57) **Abstract**

The invention concerns a hybrid synthetase obtainable by at least one exchange of an entire amino acid activating module of a wild-type synthetase present in a fungus or a bacterium by another entire amino acid activating module of a wild-type synthetase present in the same or another fungus or bacterium.

## Description

### Introduction

Non-ribosomal, enzymatic synthesis of peptides by the multiple carrier thiotemplates present in numerous fungi and bacteria is a fascinating topic of molecular microbiology that has been intensively investigated during the last decade. The hundreds of types of peptides synthesized by this mechanism form a diverse group of bioactive natural products, such as antibiotics, antitumor and anti-viral agents, biosurfactants, enzyme inhibitors, immunosupressants and toxins, with important medical and biotechnological applications. The broad spectrum of distinct activities reflects the structural diversity of these ordinarily short, linear, branched or cyclic peptides, most of which are additionally modified: some 300 different types of amino acid residue, including acylated, D-, hydroxy-, N-methylated, glycosylated or non-protein amino acids may be incorporated into the final product¹.
All prokaryotic peptide synthetases exhibit a multi-modular structure with highly conserved features involved in the stepwise assembly of the peptide chain². This structural architecture, and the autonomous nature of the modules, a coordinated transcription and physical linkage in the multienzyme complex of which are not essential for proper assembly and activity of the peptide synthetases³, facilitates the creation of new peptide products by the exchange of modules through gene replacement⁴.

Recently, it was shown that the amino acid binding domains within the SrfAA2 and SrfAC modules of surfactin synthetase can replaced by the corresponding domains of heterologous synthetases exhibiting different specificities^{4,5}. However, despite of the fact that these heterologous minimal modules to retain their specificity for activation of their cognate substrates, the resulting hybrid peptide synthetases generally exhibit greatly reduced lipopeptide biosynthesis activities⁵. Modification of peptide synthetases by moving the carboxy-terminal intrinsic thioesterase region to the end of the internal amino acid-binding domains also provoked a substantial reduction in synthetic activity of the engineered enzyme⁶. Thus, structural changes in domain-encoding regions within modules may perturb internal domain-domain interactions important for catalytic efficiency, and renders the general applicability of this approach questionable for the creation of hybrid enzymes to produce novel peptides.

### Invention

One object of the invention concerns a hybrid synthetase obtainable by at least one exchange of an entire amino acid activating module of a wild-type synthetase present in a fungus or a bacterium by another entire amino acid activating module of a wild-type synthetase present in the same or another fungus or bacterium.

A regards the hybrid synthetase according to the invention, the bacteria may belong to Bacillus, especially B. licheniformis or B. subtilis.

Further, the hybrid synthetase according to the invention may be obtainable by at least one exchange of an entire amino acid activating module of wild-type lichenysin A synthetase present in B. licheniformis.

Further, the hybrid synthetase according to the invention may be obtainable by an exchange of the entire Glu activation module.

Further, the hybrid synthetase according to the invention may be obtainable by at least one exchange of an entire amino acid activating module by another entire amino acid activating module of a wild-type synthetase present in B. subtilis.

Further, the hybrid synthetase according to the invention may be obtainable by an exchange of the entire Glu activating module by an entire Gln activating module.

As regards the hybrid synthetase according to the invention the exchange of the entire amino acid activating module may comprise amino acid residues at the two strongly conserved extremities of the amino acid activating module.

Further, as regards the hybrid synthetase according to the invention the strongly conserved extremities may comprise the sequence (MV)HH(MVI)I(ST)DG.

Further, an object of the invention concerns a fungus strain or bacterium strain, comprising a hybrid synthetase, obtainable by at least one exchange of an entire amino acid activating module of the wild-type synthetase of the fungus or bacterium strain by another entire amino acid module of a wild-type synthetase present in the same or in another strain of the same fungus or bacterium or present in another strain of another fungus or bacterium.

Further, an object of the invention concerns a fungus strain or bacterium strain, wherein the chromosome encodes a hybrid synthetase according to the invention.

As regards the fungus strain or bacterium strain according to the invention, the chromosome enoding a hybrid synthetase according to the invention may result from an in-frame replacement of the coding region of at least one entire amino acid activating module of a wild-type synthetase present in said fungus or bacterium strain.

Further, as regards the fungus or bacterium strain according to the invention, the coding region of at least one entire amino acid activating module may enode
- the condensation domain,
- the substrate recognition and amino adenylation domain,
- the thiolation domain,
- optionally the epimerization domain and
- optionally the thioesterase domain.

Further, as regards the fungus strain or bacterium strain according to the invention, the strain may be a strain for the production of recombinant peptides.

Further, as regards the fungus strain or bacterium strain according to the invention, the wild-type synthetase of said fungus or bacterium strain is highly expressible by said fungus or bacterium strain.

Further, an object of the invention concerns a hybrid peptide, obtainable by non-ribosomal enzymatic synthesis by means of a hybrid synthetase according to the invention.

Further, an object of the invention concerns a hybrid peptide, obtainable by non-ribosomal enzymatic synthesis by means of a fungus or bacterium according to the invention.

Further, as regards the hybrid peptide according to the invention, the hybrid peptide may be a lipopeptide.

Further, an object of the invention concerns a pharmaceutical composition, comprising at least one hybrid peptide according to the invention and at least one usual carrier and/or diluent.

Further, another object of the invention concerns an agricultural or agrochemical composition, comprising at least one hybrid peptide according to the invention and at least one usual carrier and/or diluent.

Further, still another object of the invention concerns a chemical composition, comprising at least one hybrid peptide according to the invention and at least one usual carrier and/or diluent.

The inventors have therefore explored the possibility that exchange of entire amino acid
activating modules may conserve principal structural features important for domain-domain interactions, and generate hybrid enzymes with high catalytic activities.

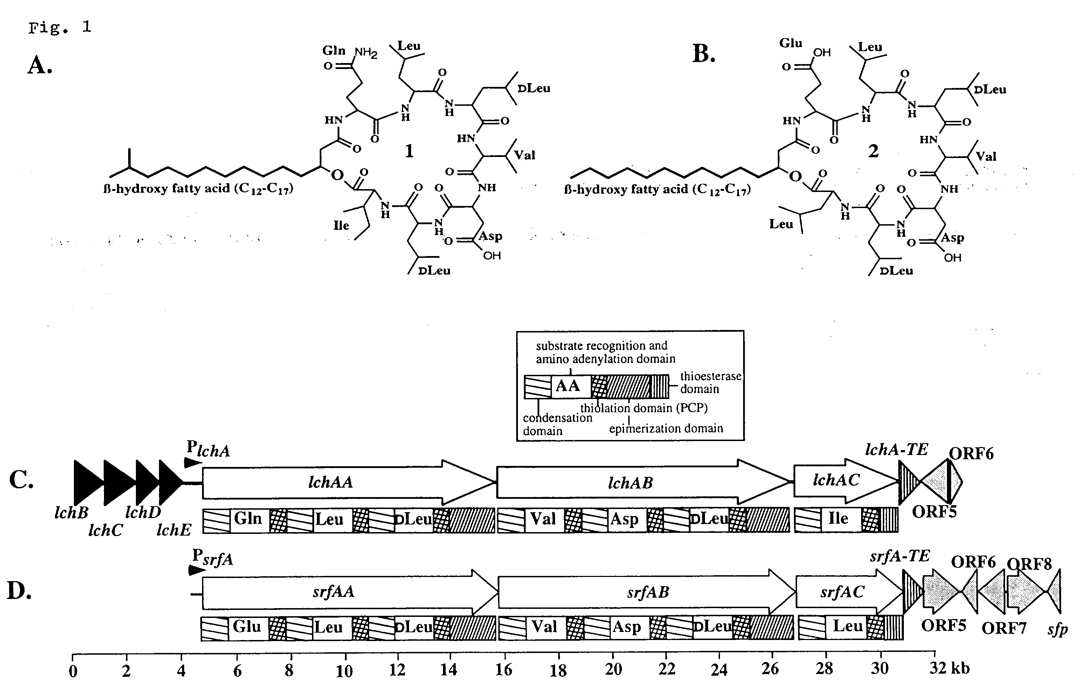

To assess the utility of whole module replacement we employed as model systems the surfactin and lichenysin A synthetases, which produce the structurally related lipopeptides, surfactin and lichenysin A, respectively (Fig.1A)⁷ and have similar modular molecular architectures (Fig.1B)⁸. Both peptides are of biotechnological interest due to their biological and surface active properties⁹, whereby lichenysin A exhibits a higher specific activity than surfactin, but is made at the levels of around only 10% of that of surfactin, and the producer strains *B.licheniformis* are not amenable to genetic manipulation^{7,9}.

In this report , we describe the construction of *B.subtilis* recombinants containing a hybrid surfactin synthetase which produce high levels of a novel lipopeptide exhibiting the activity level of lichenysin A.
Thus, the invention concerns the targeted exchange of homologous modules from two different peptide synthetases, the lichenysin A and surfactin synthetases was performed in order to form a functional hybrid enzyme and to produce a new recombinant lipopeptide. Engineered enzyme was created by in-frame replacement of coding regions of glutamate-activating module of surfactin synthetase subunit SrfAA1 with the glutamine-activating module of lichenysin A synthetase subunit LchAA1, between histidine 140 and histidine 1184, and the amino acid residues of active-site motif (HHXXXDG) within the condensation domains of SrfAA1 and SrfAA2, involved in the catalysis of nonribosomal peptide bond formation. When the lipopeptides produced by the recombinant Bacillus subtilis strains were purified and characterized, they appeared to be expressed approximately at the same level of the wild type surfactin and to be similar to lichenysin A in having glutamine residue in peptide moiety while the fatty acid profile was identical to that of surfactin.

### Discussion

Lichenysin A, produced by *B. licheniformis* strain BAS29, is a cyclic lipopeptide characterized by one of the highest biosurfactant activities thus far reported. It is also halotolerant, which makes it particularly suitable for applications in salt-containing situations, such as tertiary oil recovery. Whereas it is superior to the well known lipopeptide surfactin produced by *B. subtilis,* both in terms of its specific activity and halotolerance, lichenysin A is produced in much lower amounts, more than one order of magnitude lower, than surfactin. Although current knowledge on the expression of peptide synthetases is such that it should be possible to achieve much higher levels of synthesis of lichenysin A by genetic optimization of expression signals, all attempts to genetically manipulate *B. licheniformis* have so far been unsuccessful. Lichenysin A differs structurally from surfactin in three respects, namely qualitatively, in two constituent amino acids - it has glutamine instead of glutamate as the first residue, and isoleucine instead of leucine as the last - and quantitatively, in the composition of its lipid substituents. It is likely, however, that the Glu/Gln difference is the most relevant to the difference in activities of these two lipopeptides. Thus, in accordance with the invention, it was reasoned that if it were possible to substitute the Glu residue of surfactin by Gln residue of lichenysin A, by exchanging the genetic determinants of the correspondent modules of the peptide synthetase, a new lipopeptide would be generated with properties similar to those of lichenysin A. In accordance with the invention it was further reasoned that because the manipulations would involve entire enzyme modules, they should not result in the perturbations of intra-molecule interactions critical to enzyme activity, experienced in earlier work involving the exchange of mini- domains, and should therefore result in a hybrid lipopeptide synthesized in *B. subtilis* in amounts similar to those typical of surfactin.
The directed reprogramming of surfactin synthetase was accomplished by targeted module disruption within the *srfA* operon in the chromosome of *B*. *subtilis* OKB105, followed by replacement with the coding region of the Gln-activating module of lichenysin A synthetase. This approach involved the deletion of the chromosomal target site (Glu-activating module of SrfAA) and its replacement by the selectable Km resistance gene *aphA-3* , followed by the replacement of the *aphA-3* gene with the gene encoding Gln-activating module of LchAA, by homologus recombination events. The substrate condensation/elongation region present in all amino acid-activating modules of surfactin and lichenysin A synthetases, which is characterized by a high degree of variation compared with other functional domains within the module structure^{2,11} (and whose specific sequence was therefore assumed to be reasonably tolerant of minor sequence changes), was chosen as a site for module replacements. PCR approach for amplifying the whole module-encoding fragment was developed based on the strong conservation of the core sequence S, (MV)HH(MVI)I(ST)DG, within this region . The PCR-generated *Pml* I and *Nsi* I sites at the two extremities of amplified fragments were chosen as cloning sites, due to their presence within wild-type core sequences of several *srfA* and *lchA* modules. The cloning strategy thus minimized substitutions of amino acids in the polypeptide chain, resulting from the creation of restriction sites, which might cause conformational and adverse effects on the activity changes of hybrid synthetases. This whole module substitution approach technique, in contrast to that of the swapping of mini-modules^{4,5} allows the construction of hybrid synthetases with minimal perturbations of specific intramodular domain-domain interactions that determine the correct assembly and activity of the native peptide synthetases. The recombinant lipopeptides produced by the engineered*Bacillus subtilis* strain was expressed at approximately at the same level as that of wild type surfactin, i.e. a twelve-fourteen-fold higher yield than that of lichenysin A produced by *B.licheniformis* BNP29^{7,8}.
Structural characterization of recombinant lipopeptide showed that its fatty acid profile to be identical to that of surfactin and distinct from that of lichenysin.
This indicates that the high surface activity and halotolerance of recombinantlipopeptide are due to the glutamine residue inserted into the cyclic peptide.

In conclusion, the physico-chemical properties of the recombinant lipopeptide are indistinguishable from those of lichenysin A. The work reported here has thus three messages (a) that module exchange in peptide synthetases can create novel peptides without compromizing the catalytic activity of the sythetases, (b) that this in turn allows the reprogramming of highly active synthetase such that they synthetize high levels of novel peptides with activities identical to natural peptides that may only be poorly expressed from their normal producer strain, as in the case for lichenysin A, and (c) that of the three structural differences between surfactin and lichenysin A, namely Glu/Gln, Leu/Ile and the composition of the fatty acid substituents, it is primarily the Glu/Gln difference which is responsible for the activity differences between these two peptides. These findings open up new possibilities for the engineering of novel peptides and lipopeptides of biotechnological interest.

### Figure legends

**Figure 1.** Primary structure of lipoheptapeptides and organisation of their genetic determinants. The primary structure of lichenysin A (**A**) and surfactin (**B**), and the structural organization of the chromosomal regions containing the lichenysin A, *lchAA-C* (**C**) and surfactin, *srfAA-C* (**D**), synthetase operons are shown, as are the locations of promoters (*P*) and genes associated with these operons (genes encoding ABC-like transporter system; *lchB-E,* thioesterases, *lchA-TE* and *srfA-TE;* 4'-PP transferase, *sfp;* and genes with unknown functions, ORF5-8).

**Figure 2.** Strategy for whole-module targeted replacement of *srfAA1* in *B. subtilis.*

**Figure 3.** Analysis of the *srfAA1* locus in the constructs. PCR amplification with the CHECKF (389) and CHECKR (3581) primers was carried out with chromosomal DNA isolated from various *B. subtilis* strains used and obtained in this work. Lane A, *B. subtilis* OKB105; lane B, *B. subtilis* ΔGlu (Km^{R}, Em^{S}, Srf⁻); lane C, *B*. *subtilis* merodiploid intermediate ΔGlu/Gln (Km^{R}, Em^{R}, Srf⁻); lane D, *B. subtilis* 1D1. (Km^{S}, Em^{S}, Srf⁻, "Lch"⁺).

**Figure 4.** Physico-chemical properties of native and recombinant lipoheptapeptides.
(**A**) TLC of lipopeptides in an alkaline solvent system and their corresponding FAB⁻ MS spectra. Lane 1, surfactin; lane 2, lichenysin A; lane 3, recombinant lipopeptide 1D1.
(**B**) Surface tension characteristics of surtactin (open squares) and lipopeptide 1D1. (open circles) as function of NaCl concentration.

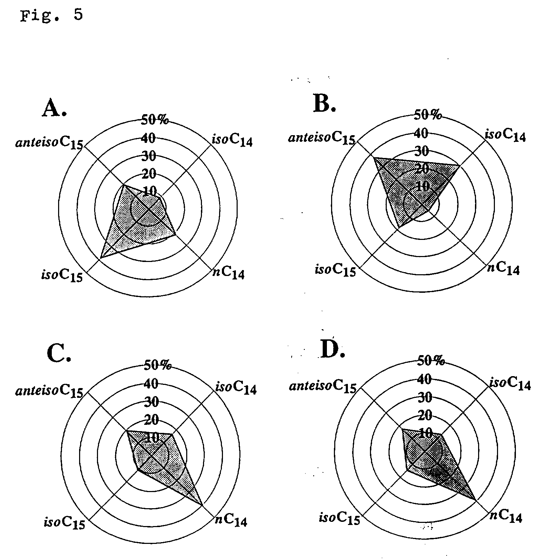

Figure 5. Relative abundances of the four major β-hydroxy fatty acid series constituting the lipid tails of the lipopeptides: **A:** native lichenysin A from *B. lichenifonnis;* **B:** wild type lichenysin B⁷ ; **C:** native surfactin; and **D:** recombinant lipopeptide 1D1.

### Experimental protocol

The bacteria employed in this study were the surfactin-producing *B. subtilis* strain OKB 105 and its isogenic *ΔsrfAA1, ΔsrfAB2, ΔsrfAA1*(*lchAA1*) and Δ*srfAB2*(*lchAB2*) mutants, which were routinely grown at 30°C in CMM minimal medium⁷ or Luria-Bertani¹² agar, supplemented where appropriate with 100 µg of kanamycin (Km) and 20 µg of erythromycin (Em) per ml.

PCR amplification of DNA fragments was used to generate the terminal restriction sites needed for subsequent cloning and was performed using OKB 105 and BNP29 chromosomal DNA as the template, TaqPlus Long (Stratagene) polymerase and following the manufacturer protocol. The sequences of the oligonucleotide primers were as follows (bracket, location from the start codon of corresponding synthetase subunit; underlined, modified sequence; bold, created restriction site): 
F-PSrf-1-Kpn I (-528), 5'-GCGCGGTACCCATAGTCATGTAAAG-3'; R-SrfAA1-Eco RI
(455), 5'-CCCGACAGAATTCATGGACATACCATC-3';     F-SrfAA2-Eco RI/Nsi I
(3534), 5'-CTGGAATTCGACATGCATCATTTGATTTCTGACGGTGTATCC -3'; R-
SrfAA2 (4282), 5'-CGTATTCGCCGCTTGCTCCTC-3'; F-LchAA1-Eco RV (411), 5'-
CATGATATCATTTCCGATGGTATTTCGATGACG-3'; R-LchAA2 (3645), 5'-
CGGCTCCGGCAGTTCTTCCC-3'.
 The PCR was carried out in a Perkin-Elmer Thermal Cycler 480 (Ueberlingen, Germany), and products were purified with QIA quick-spin PCR purification kit as described by the manufacturer.

Standard procedures were used for DNA digestion with restriction enzymes, cloning of DNA fragments and preparation of recombinant plasmid DNA¹². Several steps were used to disrupt the region within *srfA* operon encoding the first domain. After amplification with the primers F-PSrf-1-Kpn I and R-SrfAA1-Eco RI, the P*srf*A-5'*srfAA1* fragments obtained were digested with *Kpn I* and *Eco* RI, and ligated into pBluescript II SK+ (Stratagene), digested with the same enzymes. The resulting plasmid was digested with *Eco* RI and *Xma* I and ligated with the *3'srfAA2* fragments obtained after amplification of OKB105 chromosomal DNA with F-SrfAA2-Eco RI/Nsi I + R-SrfAA2 primers and digested with the same enzymes. The plasmid obtained, designated pSRF-1D, was digested with *Pml* I and *Nsi* I and ligated with an amplified *aphaA-3* gene¹⁰ (Km^{R}) fragment flanked by PCR-generated *Pml* I and *Nsi* I sites at the 5'- and the 3'-termini, respectively. This produced the disruption plasmid pSTEP-Δ1D. In order to generate the module-substitution plasmid, the 3.1-kb DNA fragment encoding LchAA1 module was amplified, digested with *Pml* I and *Nsi* I and ligated into the same sites of pSRF-1D to produce pSRF1D1. This was subsequently digested with *Kpn* I and *Xma* I, and the ca. 5.0-kb fragment generated was introduced between *Kpn* I and *Xma* I sites of the temperature-sensitive suicide shuttle vector pAUL-A (Em^{R})¹³ to produce the plasmid pAUL-A1/1. All intermediate plasmids containing fragments generated by PCR amplification were sequenced, using universal, *srfA-* and *lchA*-derived primers, to confirm the integrity of the sequences. The pAUL-A1/1 plasmid was checked for in-frame module replacement by sequencing of the PCR product generated by primers _{CHECK}F (389), 5'-GAAGTGTGGTTTTACGCAAA-3' and _{CHECK}R (3581), 5'-GCCAATGGATACACCGTCAG-3' .

Transformation of *B. subtilis* cells was performed, as. described previously^{l4}. Homologous recombination and integration of the pAUL-A derivative into the chromosome of the strain, previously transformed with the linear form of disruption plasmid, and subsequent spontaneous excision of the integrated plasmid through intramolecular homologous recombination, was carried out as described previously¹⁵. Lipopeptide purification, thin-layer (TLC), and high performance liquid chromatography (HPLC), mass-spectrometry and protein sequencing was performed as described previously⁷. The chemical composition of recombinant lipopeptide was established after total hydrolysis of the product and subsequent gas chromatography-mass spectrometry (GC-MS) analysis of the β-hydroxy fatty and amino acids as corresponding *C*-methyl β-trimethylsilyl (FAME/TMS) and *N*-trifluoroacetyl *C*-*n*-butyl esters, respectively⁷.

### Results

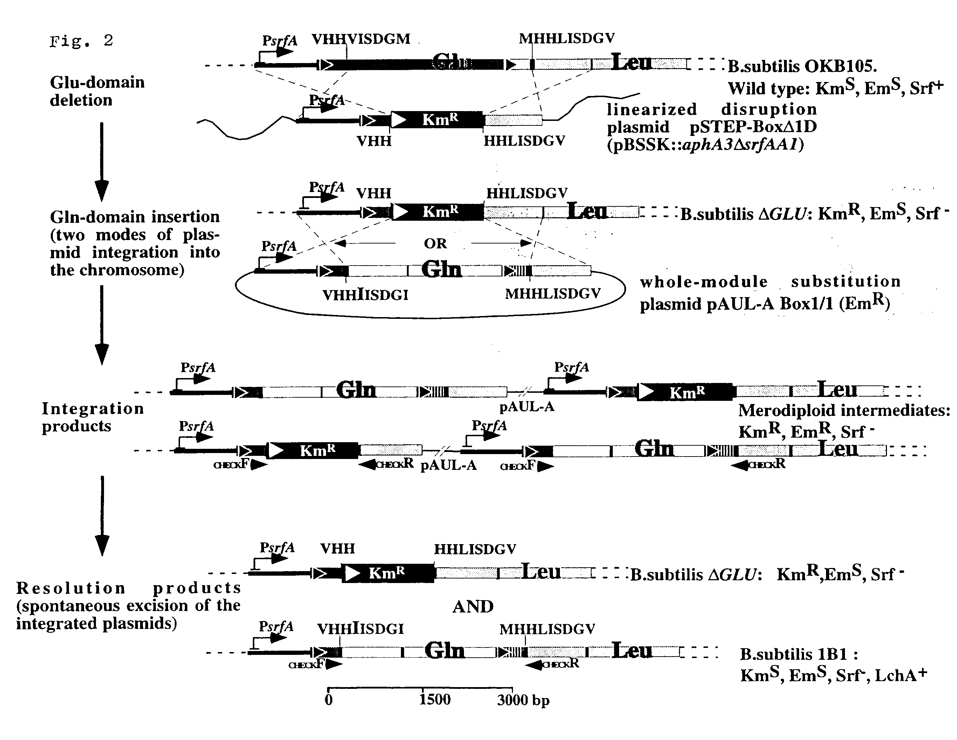

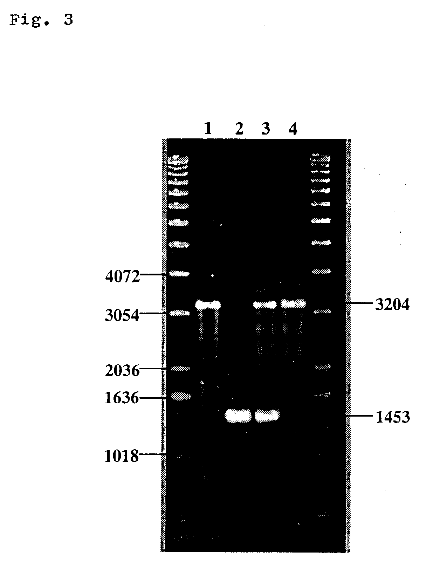

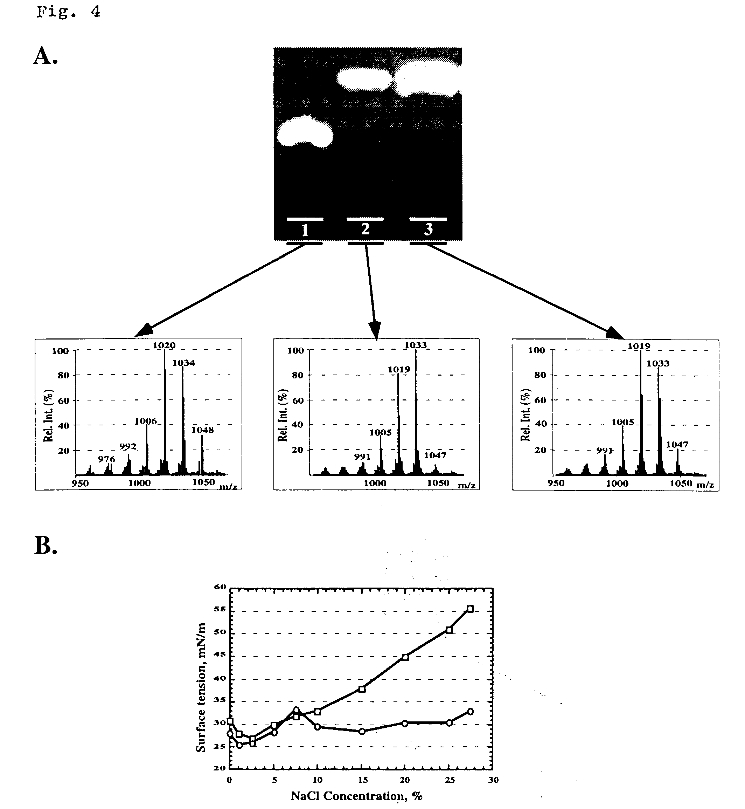

**Construction of a hybrid surfactin synthetase.** A deletion within the surfactin synthetase locus *srfA* was generated by the strategy shown in Fig. 2, and described in detail in Experimental protocol. First, the 3.13-kb fragment extending from nucleotide 420 to 3550 in srfAORF1 was replaced by a 1.4-kb fragment carrying a kanamycin resistance *aphA-3* gene¹⁰. Restriction and sequencing analysis of the relevant PCR-amplified product, using the _{CHECK}F and _{CHECK}R primers, revealed kanamycin-resistance gene at the chromosomal location of the 3.1-kb of *srfAA*-coding region in Km^{R} clones deficient in surfactin production (Fig. 3). Second, the module-substitution plasmid, pAUL-A1/1 was introduced by transformation into the deletion mutant in order to replace the *aphA-3* with the desired lichenysin A module sequence by marker exchange. Spontaneous excision of integrated plasmids from the merodiploid intermediates (Em^{R}, Km^{R}) by intramolecular homologous recombination (Fig. 2) was allowed to occur under nonselective conditions. Erythromycin-sensitive cells (Em^{S}) were selected and screened for kanamycin sensitivity.10% - 17% of Em^{S} transformants had desired phenotype (Em^{S}, Km^{S}), and these were tested on blood agar plates for haemolytic activity.
Halo-positive clones (60% - 89% of Em^{S}, Km^{S} transformants) were analysed for correctness of in-frame substitution within hybrid genes, by direct PCR amplification of chromosomal DNA from these double recombinants, using the _{CHECK}F and _{CHECK}R primers complementary to the chromosomal regions located upstream or downstream of the integration sites, and sequencing (Fig. 3). This confirmed the expected substitution within the hybrid module of *srfA* operon in the chromosome of *B. subtilis* 1D1. (for reference see AoXXXX).
**Physico-chemical characterization of the hybrid surfactant**. The recombinant lipopeptide produced by the hybrid synthetase was extracted from the culture broth of strain 1D1, purified, and analysed by TLC using a chloroform:methanol:28%NH₄OH (65:25:8, v/v/v) solvent system⁷. The mobility index (R_{*f*}) of the hybrid lipopeptide in the TLC system employed is like that of wild-type lichenysin A and distinctly different from that of surfactin (Fig. 4A). The negative FAB ionization MS of lichenysin A and recombinant lipopeptide were identical and showed deprotonated molecular ions [M-H]⁻ at *m/z*.991, 1005, 1019, 1033 and 1047 (Fig. 4A), while the structurally similar surfactin yielded in the same ionization mode major peaks that are 1 Dalton more, namely at *m/z* 1006, 1020 and 1034, reflecting the molecular mass difference between glutamine and glutamate⁸. These data, together with the lower polarity of recombinant lipopeptidc 1D1 compared to that of surfactin, clearly indicate the replacement of the original Glu-1 by a Gln-1-residue, as deduced from the sequence of lichenysin A.
**Surface activity of the recombinant lipopeptide**. The recombinant lipopeptide exhibited a high surface activity, in that it decreased the surface tension of water from 72 mN/m to 25.5 mN/m, and achieved a critical micelle concentration (CMC) at concentrations as low as 13.5 µM, which are similar to that of native lichenysin A (12µM) and substantially lower than that of surfactin (20µM). Moreover, in contrast to surfactin whose activity is progressively inhibited by increasing salt concentrations, the recombinant lipopeptide is not inhibited by NaCI concentrations up to 25% (Fig.4B). Such a halotolerance was previously described for lichenysin A, which is produced by thermo- and halotolerant *B. lichenformis*^{7,9}.
**Lipid composition of recombinant lipopeptide**. To investigate the lipid moieties of recombinant lipopeptide, the β-hydroxy fatty acids were released by hydrolysis and analysed as Me/TMS derivates by GC-MS. The resulting spectrum showed that four β-hydroxy acids, namely isomyristic, myristic, 13-methylmyristic and 12-methyltetradecanoic acids, occur in the lipid moieties as major residues, and make up about 88% of the total fatty acids present in lipopeptide. The distribution of these fatty acids in the recombinant lipopeptide clearly corresponds to that of native surfactin, and not of wild-type lichenysin A (Fig. 5).
**Biosynthesis levels of the recombinant lipopeptide**. Analysis of the specific yield (nmol of product per mg of dry biomass), of recombinant lipopeptide from *B. subtilis* revealed yields similar to that of native surfactin producted by *B. subtilis* OKB105, namely around twelve- fold higher than yields of native lichenysin A by strain *B. licheniformis* BNP29 (Table 1).

**Table 1.**

| Lipopeptide production of parent and recombinant bacilli. | | |
|---|---|---|
| Strain / description | Dry biomass, g/l | Lipopeptide yield, µM/g |
| *B.subtilis* OKB105 / Wild type surfactin | 1.92 ± 0.05 | 792 ± 16 |
| Recomb. 1D1 / F(*Psrf*A-*lchAA1*[*Gln*]-*srfAA2* | 1.89 ± 0.03 | 730 ± 54 |
| *B.licheniformis* BNP29/Wild type lichenysin A | 1.85 ± 0.04 | 58 ± 7 |

### References

1. Kleinkauf, H., and von Dohren, H. 1997. Applications of peptide synthetases in the synthesis of peptide analogues. Acta Biochim. Pol.**44**:839-847.
2. Marahiel, M.A. 1997. Protein templates for the biosynthesis of peptide antibiotics. Chem. Biol. **4**:561-567.
3. Guenzi, E., Galli, G., Grgurina, I., Pace, E., Ferranti, P., and Grandi, G. 1998. Coordinate transcription and physical linkage of domains in surfactin synthetase are not essential for proper assembly and activity of the multienzyme complex. J. Biol. Chem. **273**:14403-14410.
4. Stachelhaus, T., Schneider, A., and Marahiel, M.A. 1995. Rational design of peptide antibiotics by targeted replacement of bacterial and fungal domains. Science **269**:69-72.
5. Schneider, A., Stachelhaus, T., and Marahiel, M.A. 1998. Targeted alteration of the substrate specificity of peptide synthetases by rational module swapping. Mol. Gen. Genet. **257**:308-318.
6. de Ferra, F., Rodriguez, F., Tortora, O., Tosi, C., and Grandi, G. 1997. Engineering of peptide synthetases. Key role of the thioesterase-like domain for efficient production of recombinant peptides. J. Biol. Chem. **272**:25304-25309.
7 Yakimov, M.M., Timmis, K.N., Wray, V., and Fredrickson, H.L. 1995. Characterization of a new lipopeptide surfactant produced by thermotolerant and halotolerant subsurface *Bacillus lichenifonnis* BAS50. Appl. Environ. Microbiol. 61:1706-1713.
8. Yakimov, M.M., Kroger, A., Slepak, T.N., Giuliano, L., Timmis, K.N., and Golyshin, P.N. 1998. A putative lichenysin A synthetase operon in *Bacillus licheniformis*: initial characterization Biochim. Biophys. Acta **1399**:141-153.
9. Yakimov, M.M., Amro, M.M., Bock, M., Boseker, K., Fredrickson, H.L., Kessel, D.G., and Timmis, K.N. 1997. The potential of *Bacillus licheniformis* strains for in situ enhanced oil recovery. J. Petrol. Sci. Engineer. **18**:147-160.
10. Trieu-Cuot, P., Gerbaud, G., Lambert, T. and Courvalin, P. 1985. In vivo transfer of genetic information between gram-positive and gram-negative bacteria. EMBO J. **4**:3583-3587.
11. Stachelhaus, T., Mootz, H.D., Bergendahl, V., and Marahiel, M.A. 1998. Peptide bond formation in nonribosomal peptide biosynthesis. Catalytic role of the condensation domain. J. Biol. Chem. **273**:22773-22781.
12. Sambrook, J.,Fritsch, E.F., and Maniatis, T. 1989. Molecular cloning: a laboratory manual. 2nd ed. Cold Spring Harbor Laboratory Press, Cold Spring Harbor, N. Y.
13. Schaferkordt, S., and Chakraborty, T. 1995. Vector plasmid for insertional mutagenesis and directional cloning in *Listeria* spp. BioTechniques **19**:720-725.
14. Yakimov, M.M., and Golyshin, P.N. 1997. ComA-dependent transcriptional activation of lichenysin A synthetase promoter in *Bacillus subtilis* cells. Biotechnol.Prog. **13**:757-761
15. Guzman, C.A., Rhode, M., Chakraborty, T., Domann, E., Hudel, M., Wehland, J., and Timmis, K.N. 1995. Interaction of *Listeria monocytogenes* with mouse dendric cells. Infect. Immun. **63**:3665-3673.

## Claims

1. Hybrid synthetase obtainable by at least one exchange of an entire amino acid activating module of a wild-type synthetase present in a fungus or a bacterium by another entire amino acid activating module of a wild-type synthetase present in the same or another fungus or bacterium.

2. Hybrid synthetase according to claim 1, wherein the bacteria belong to Bacillus, especially B. licheniformis or B. subtilis.

3. Hybrid synthetase according to claim 1 or 2, obtainable by at least one exchange of an entire amino acid activating module of wild-type lichenysin A synthetase present in B. licheniformis.

4. Hybrid synthetase according to any of the preceding claims, obtainable by an exchange of the entire Glu activation module.

5. Hybrid synthetase according to any of the preceding claims, obtainable by at least one exchange of an entire amino acid activating module by another entire amino acid activating module of a wild-type synthetase present in B. subtilis.

6. Hybrid synthetase according to claim 5, obtainable by an exchange of the entire Glu activating module by an entire Gln activating module.

7. Hybrid synthetase according to any of the preceding claims, wherein the exchange of the entire amino acid activating module comprises amino acid residues at the two strongly conserved extremities of the amino acid activating module.

8. Hybrid synthetase according to claim 7, wherein the strongly conserved extremities comprise the sequence (MV)HH(MVI)I(ST)DG.

9. Fungus strain or bacterium strain, comprising a hybrid synthetase, obtainable by at least one exchange of an entire amino acid activating module of the wild-type synthetase of the fungus or bacterium strain by another entire amino acid module of a wild-type synthetase present in the same or in another strain of the same fungus or bacterium or present in another strain of another fungus or bacterium.

10. Fungus strain or bacterium strain, wherein the chromosome encodes a hybrid synthetase according to any of claims 1 to 8.

11. Fungus strain or bacterium strain according to claim 10, wherein the chromosome enoding a hybrid synthetase according to any of claim 1 to 8 results from an in-frame replacement of the coding region of at least one entire amino acid activating module of a wild-type synthetase present in said fungus or bacterium strain.

12. Fungus or bacterium strain according to claims 10 or 11, wherein the coding region of at least one entire amino acid activating module enodes
- the condensation domain,
- the substrate recognition and amino adenylation domain,
- the thiolation domain,
- optionally the epimerization domain and
- optionally the thioesterase domain.

13. Fungus strain or bacterium strain according to claim 9, 10, 11 or 12, wherein the strain is a strain for the production of recombinant peptides.

14. Fungus strain or bacterium strain according to claim 9, 10, 11, 12 or 13, wherein the wild-type synthetase of said fungus or bacterium strain is highly expressible by said fungus or bacterium strain.

15. Hybrid peptide, obtainable by non-ribosomal enzymatic synthesis by means of a hybrid synthetase according to any of claims 1 to 8.

16. Hybrid peptide, obtainable by non-ribosomal enzymatic synthesis by means of a fungus or bacterium according to claim 9 or 14.

17. Hybrid peptide according to claim 15, wherein the hybrid peptide is a lipopeptide.

18. Pharmaceutical composition, comprising at least one hybrid peptide according to claim 15, 16 or 17 and at least one usual carrier and/or diluent.

19. Agricultural or agrochemical composition, comprising at least one hybrid peptide according to claim 15, 16 or 17 and at least one usual carrier and/or diluent.

20. Chemical composition, comprising at least one hybrid peptide according to claim 15, 16 or 17 and at least one usual carrier and/or diluent.
